# EUROPEAN PATENT SPECIFICATION

(11) **EP 2 227 286 B1**
(45) Date of publication and mention of the grant of the patent: **19.04.2017**
(21) Application number: 08853959.8
(22) Date of filing: 28.11.2008
(51) Int. Cl.: A61M 37/00, A61M 31/00, A61K 9/00, A61M 5/20

(54) **A DELIVERY DEVICE FOR ADMINISTERING AN ACTIVE SUBSTANCE TO A SUBJECT**
ABGABEVORRICHTUNG ZUR VERABREICHUNG EINER WIRKSUBSTANZ AN EINE PERSON
DISPOSITIF DE DISTRIBUTION POUR L'ADMINISTRATION D'UNE SUBSTANCE ACTIVE À UN SUJET

(30) Priority: 28.11.2007 IE 20070861; 04.11.2008 IE 20080884
(43) Date of publication of application: 15.09.2010
(73) Proprietor: Janisys Limited, Galway (IE)
(72) Inventor: O'DEA, John, Galway (IE); BAMBURY, Eoin, County Meath (IE)
(74) Representative: Gorman, Francis Fergus
(86) International application number: PCT/IE2008/000115
(87) International publication number: WO 2009/069112

(56) References cited:
- EP-A- 1 216 721
- EP-A- 1 844 763
- WO-A-01/36037
- WO-A-2004/026281
- WO-A-2004/084857
- WO-A-2006/015299
- WO-A-2008/008557
- WO-A1-2009/047555
- US-A- 5 542 920
- US-A- 5 782 799
- US-A1- 2005 251 088

## Description

The present invention relates to a delivery device for administering an active substance to a subject.

Delivery devices for delivering an active substance to a subject are known. One type of delivery device which Is currently gaining acceptance is commonly referred to as a micro-delivery device. Such micro-delivery devices are particularly suitable for delivering an active substance transdermally, transcutaneously and intradermally, and comprise a laminated structure, which typically comprises three or four layers which are separated by respective membranes. A first layer which is located between a second layer and a third layer Is provided with a plurality of first chambers for storing the active substance to be administered transdermally to the subject The first chambers typically are formed by bores extending through the first layer and are arranged in a matrix in the first layer and sealed by respective first and second membranes located at respective opposite major surfaces of the first layer.

The second layer typically comprises a plurality of second chambers formed by bores extending through the second layer and arranged in a matrix and aligned with the corresponding first chambers. The first membrane is located between the first and second layers for sealably isolating the first and second chambers from each other. A driving medium is located in each of the second chambers for urging the first membrane into the first chambers for discharging the active substance from the corresponding first chambers. Typically, the driving medium comprises an expandable medium which expands in response to an increase in temperature.

The third layer is located on the opposite side of the first layer to that of the second layer and comprises a plurality of micro-needles extending therefrom for penetrating the skin of a subject. A bore extends through each micro-needle for accommodating the active substance therethrough from the corresponding first chamber. The second membrane is located between the first layer and the third layer, which sealably isolates the first chambers from the bores of the respective micro-needles. The second membrane is of a burstable material which bursts in response to an increase in pressure in the first chambers resulting from expansion of the first membrane into the first chambers for in turn communicating the first chambers with the bores through the corresponding micro-needles for delivering the active substance transdermally to the subject.

In such micro-delivery devices a fourth layer is provided which comprises a plurality of heating elements which are formed on the fourth layer for raising the temperature of the driving medium. The fourth layer with the heating elements thereon may be secured directly to the third layer, or secured to the third layer through a third membrane which sealably isolates the second chambers from the corresponding heating elements. By appropriately activating the heating elements to raise the temperature of the driving medium, the driving medium expands, thereby urging the first membrane into the first chambers and raising the pressure of the active substance in the first chambers. The rise in pressure in the first chambers results in the second membrane bursting at locations adjacent the first chambers, thereby communicating the first chambers with the bores in the corresponding micro-needles. The action of the first membrane on the active substance urges the active substance from the first chambers through the bores in the micro-needles, and in turn transdermally into the subject.

Typically the heating elements are activatable individually sequentially or in groups sequentially for delivering the active substance to the subject in specific doses to comply with a predefined treatment regime. Such a treatment regime may require a dose of the active substance to be administered to the subject three times per day during a treatment period, which could be a period of five days or more. This requires the micro-delivery device to be attached to the subject for the duration of the treatment period with the micro-needles continuously penetrating the skin of the subject. This is undesirable, since it can lead to irritation and infection of the skin, which leads to and in many cases considerable discomfort.

There is therefore a need for a delivery device for administering an active substance to a subject which addresses this problem.

U.S. Patent Specification No. 5,542,920 of Cheikh discloses a device for administering a medicament in solid form subcutaneously to a subject. The medicament in solid form terminates in a pointed tip for penetrating the skin of the subject. A plurality of the medicaments in solid form are stored in a magazine, and are sequentially alignable with a bore in a housing. A high pressure gas discharges the medicaments through the bore as they are sequentially aligned therewith, and in turn urges the medicaments subcutaneously through the skin of a subject.

U.S. published Patent Application Specification No. 2005/0251088 of Kwon discloses an array of porous micro-perforators mounted on a carrier plate. The porous micro-perforators are impregnated with a drug so that when the micro-perforators penetrate through the skin of a subject, the drug is absorbed from the perforators. The micro-perforators may also be of a dissolvable material, which would dissolve in the subject.

PCT published Application Specification No. WO 2004/084857 of Browning comprises a device for delivering a drug into the uterine myometrium in females and into the prostate gland of males. The delivery device comprises an elongated cylindrical body terminating in a semi-sharp point 10 which extends from a flat plate. The body of the device is implanted in the tissue of the myometrium.

Published PCT Application Specification No. WO 2004/026281 of Coppeta et al discloses a device which provides for controlled release of drugs to a subject The device comprises a substrate with a plurality of reservoirs formed therein. Each reservoir contains a drug and an osmotic pressure generating agent for driving the drug from the reservoir. The osmotic pressure generating agent is separated from the drug in the reservoir, and is activated to pressurise the drug by the controlled admission of a suitable fluid into the reservoir through a semi-permeable membrane.

U.S. Patent Specification No. 5,782,799 of Jacobson, et al discloses an automatic micro-delivery device for dosing of drugs to a subject The micro-delivery device may be implanted in or otherwise administered to an animal or human. The micro-delivery device comprises a plurality of compartments each containing at least one drug so that a plurality of doses of drugs are held within the device. The micro-delivery device is programmable for selective release doses of the drug or drugs to provide an efficacious dosing pattern.

PCT Published Application Specification No. WO 2008/015299 of Santini et al discloses a transdermal delivery device for administering a pharmaceutical agent to a patient. The device comprises a substrate, a plurality of discrete reservoirs formed in the substrate which store one or more pharmaceutical agents. Discrete reservoir caps dose the reservoirs. A control means is provided for actuating release of one or more of the pharmaceutical agents from one or more of the reservoirs by disintegrating or permeabilizing the reservoir caps.

PCT Published Application Specification No. WO 2009/047555 of Kirby published on April 16, 2009 discloses a needleless device for delivering a plurality of microimplants through a biological barrier, for example, the skin of a subject. The device of Kirby comprises a microimplant retaining layer which comprises a plurality of recesses in which respective ones of the microimplants are located. A guide layer having a plurality of guide channels is located adjacent the microimplant retaining layer with the guide channels aligned with the recesses in the microimplant retaining layer. Micropistons located in the guide channels of the guide layer are aligned with corresponding ones of the microimplants in the microimplant retaining layer for urging the microimplants through the microimplant retaining layer and in turn transdermally through the skin of the subject.

The present invention is directed towards providing a delivery device for administering an active substance to a subject.

According to the invention there is provided a delivery device for administering an active substance to a subject in the form of a solid form structure, the device comprising a first housing having a plurality of first chambers located therein adapted to accommodate respective ones of the solid form structures, a second housing having a plurality of second chambers, and a plurality of urging means located in the respective second chambers for urging the solid form structures through the first chambers to penetrate the skin of the subject, wherein a first membrane is located between the first and second chambers for sealably isolating each first chamber from the second chambers, and the first membrane is deformable into each first chamber under the action of the corresponding urging means for urging the corresponding solid form structure through the first chamber.

In one embodiment of the invention the first housing comprises a plurality of solid form structures, one solid form structure being located in each first chamber.

In one embodiment of the invention the first chambers are configured in a matrix.

Preferably, the maximum transverse cross-sectional dimension of each first chamber lies in the range of 0.1 mm to 2mm. Advantageously, the axial length of each first chamber lies in the range of 0.1mm to 2mm.

Preferably, the first housing comprises a first plate member defining opposite first and second major surfaces, and each first chamber is formed by a corresponding first bore extending through the first plate member from the first major surface thereof to the second major surface. Preferably, the first plate member is of thickness in the range of 0.1 mm to 2mm. Advantageously, each first bore is of circular transverse cross-section.

Advantageously, one second chamber is provided corresponding to each first chamber, the second chambers being aligned with the respective corresponding first chambers.

Preferably, the maximum transverse cross-sectional dimension of each second chamber lies in the range of 0.1 mm to 2mm.

In one embodiment of the invention the second housing comprises a second plate member having opposite first and second major surfaces, and each second chamber is formed by a corresponding second bore extending through the second plate member from the first major surface thereof to the second major surface. Preferably, the second plate member is of thickness in the range of 0.1 mm to 2.0mm. Advantageously, each second bore Is of circular transverse cross-section.

Advantageously, the second chambers are configured In a matrix.

Preferably, the first membrane comprises a material impermeable to the urging means. Advantageously, the first membrane comprises a material impermeable to the active substance.

Preferably, the first membrane is of an expandable material.

In one embodiment of the invention a plurality of pairs of interengageable complementary formations are provided for locating the first and second plate members relative to each other with each first chamber aligned with the corresponding second chamber, one of the interengageable complementary formations of each pair thereof being located on the second major surface of the first plate member adjacent a corresponding of the first chambers and the other one of the interengageable complementary formations of each pair thereof being located on the first major surface of the second plate member, the interengageable complementary formations of the respective pairs thereof being co-operable with each other for entrapping the first membrane therebetween. Preferably, the interengageable complementary formation of each pair thereof located on the first plate member extends around the corresponding one of the first chambers. Advantageously, the interengageable complementary formation of each pair thereof located on the first plate member extends completely around the corresponding one of the first chambers. Preferably, the interengageable complementary formation of each pair thereof located on the second plate member extends around the corresponding one of the second chambers. Advantageously, the interengageable complimentary formation of each pair thereof located on the second plate member extends completely around the corresponding one of the second chambers.

In one embodiment of the invention one of the interengageable complementary formations of each pair thereof comprises a projection extending from the corresponding one of the first and second plate members. Preferably, the projection comprises an annular projection. Advantageously, the other one of the interengageable complementary formations of each pair thereof comprises a recess extending into the corresponding one of the first and second plate members for engaging the corresponding projection extending from the other one of the first and second plate members. Preferably, the recess is configured as an annular recess.

In one embodiment of the invention one pair of interengageable complementary formations is provided corresponding to each first chamber.

In another embodiment of the invention a second membrane is provided for sealably closing each first chamber adjacent the pointed tip of the corresponding solid form structure. Preferably, the second membrane is adapted to be penetrable by the pointed tip of each solid form structure.

In another embodiment of the invention at least one activating means is provided for activating the urging means to urge at least one of the solid form structures through the corresponding first chamber to penetrate the skin of a subject. Preferably, a plurality of activating means are provided, each activating means being provided for activating the urging means in a corresponding one of the second chambers.

Advantageously, the respective activating means are aligned with corresponding ones of the second chambers. Advantageously, the activating means are configured in the form of a matrix.

In one embodiment of the invention each activating means comprises a heating means. Preferably, each heating means comprises an electrically powered heating means. Advantageously, each heating means comprises a thin film resistor.

In a further embodiment of the invention a third membrane is located between the activating means and the at least one second chamber for sealably closing the second chamber.

In a still further embodiment of the invention a plurality of openings are formed through the third membrane adjacent respective corresponding ones of the second chambers, and each opening is sealably closed by a corresponding heat conducting element for conducting heat to the urging means in the corresponding second chamber. Preferably, the third membrane comprises a heat insulating material.

In one embodiment of the invention each urging means comprises an expandable driving substance.

In another embodiment of the invention the driving substance is in the form of a liquid, the liquid being responsive to one of temperature change and chemical activation for converting from a liquid phase to a gaseous phase for urging the solid form structure through the first chamber.

In another embodiment of the invention the driving substance comprises a solid responsive to one of temperature change and chemical activation for transitioning directly from a solid phase to a gaseous phase for urging the solid form structure through the first chamber. Preferably, the driving substance comprises Azobisisobutylonitrile (AIBN).

In another embodiment of the invention the driving substance comprises a plurality of gas filled microspheres responsive to temperature change for expansion thereof for urging the solid form structure through the first chamber. Preferably, the driving substance comprises gas filled microspheres sold under the Trade Mark EXPANCEL.

In a further embodiment of the invention the driving substance comprises a porous material, the pores of which are gas filled,

In one embodiment of the invention the porous material is responsive to temperature change for expansion thereof.

In another embodiment of the invention the gas In the porous material is responsive to temperature change for expanding out of the porous material.

In a further embodiment of the invention the porous material of the driving substance comprises a porous polymer material.

The advantages of the invention are many. A particularly important advantage of the invention is that the skin of the subject is not continuously penetrated by a plurality of micro-needles, as has been the case with such devices known heretofore. Thus, the risk of irritation, infection and discomfort to the subject is minimised, and in most cases is eliminated. Since the active substance is delivered to the subject by urging the solid form structures into penetrating engagement with the skin of the subject, the skin of the subject is only penetrated by those solid form structures which are urged out of the first chambers. Thus, where the solid form structures are provided to be of a biodegradable material, the solid form structures dissolve Into the skin of the subject, and once dissolved, no longer penetrate the skin to cause irritation, discomfort and possible infection. It is envisaged, in general, that each solid form structure would be of a material which would biodegrade substantially simultaneously as the last of the active substance In the solid form structure Is being released into the subject. It is envisaged that the release rate of the active substance from each solid form structure and the dissolve rate of each solid form structure could be substantially snatched, and would be such that the active substance would be released at a relatively constant rate over the period while the solid form structure is dissolving.

A further advantage of the invention is that the solid form structures containing the active substance are stored within the first chambers which are sealed by the first and second membranes, and accordingly, the solid form structures can be stored in sterile conditions.

The invention will be more clearly understood from the following description of some preferred embodiments thereof, which are given by way of example only, with reference to the accompanying drawings which are not to scale, in which:
Fig. 1 is a perspective view of a delivery device according to the invention for administering an active substance to a subject,
Fig. 2 is an exploded perspective view of the delivery device of Fig. 1,
Fig. 3 is a perspective view of the delivery device of Fig. 1 illustrated with portions of the device in a different position to that of Fig. 1,
Fig. 4 is a transverse cross-sectional side elevational view of the delivery device of Fig. 1,
Fig. 5 is a view similar to Fig. 4 illustrating the delivery device of Fig. 1 in use,
Fig. 6 is a perspective view or an injectable element for use in the delivery device of Fig. 1,
Fig. 7 is an enlarged transverse cross-sectional view of a detail of the delivery device of Fig. 1,
Fig. 8 is a perspective view of another detail of the delivery device of Fig. 1,
Fig. 9 is a top plan view of a detail of a portion of the delivery device of Fig. 1,
Fig. 10 is a transverse cross-sectional view of the detail of Fig. 9 of the delivery device of Fig. 1,
Fig. 11 is a block representation of an electronic circuit of the delivery device of Fig. 1,
Fig. 12 is a perspective view similar to Fig. 1 of a delivery device according to another embodiment of the invention for administering an active substance to a subject,
Fig. 13 is a transverse cross-sectional side elevational view similar to Fig. 4 of the delivery device of Fig. 12,
Fig. 14 is a perspective view similar to Fig. 1 of a delivery device according to another embodiment of the invention for administering an active substance to a subject,
Fig. 15 is a transverse cross-sectional side elevational view of a portion of the delivery device of Fig. 14,
Fig. 16 is a perspective view of another portion of the delivery device of Fig. 14,
Fig. 17 is a transverse cross-sectional view of a detail of the portion of Fig. 16 of the delivery device of Fig. 14,
Fig. 18 is a perspective view of another portion of the delivery device of Fig. 14,
Fig. 19 is a transverse cross-sectional side elevational view of a detail of the portion of Fig. 18 of the delivery device of Fig. 14,
Fig. 20 is a perspective view similar to Fig. 1 of a delivery device according to a further embodiment of the invention for administering an active substance to a subject,
Fig. 21 is a transverse cross-sectional side elevational view of a portion of the delivery device of Fig. 20,
Fig. 22 is a perspective view of another portion of the delivery device of Fig. 20,
Fig. 23 is a transverse cross-sectional side elevational view of a detail of the portion of Fig. 22 of the delivery device of Fig. 20,
Fig. 24 is a perspective view of another portion of the delivery device of Fig. 20,
Fig. 25 is a transverse cross-sectional side elevational view of a detail of the portion of Fig. 24 of the delivery device of Fig. 20,
Fig. 26 is a transverse cross-sectional side elevational view similar to Fig. 4 of a delivery device according to another embodiment of the invention, and
Fig. 27 is a perspective view of an injectable element for use in the delivering device of any of
Figs. 1 to 5 and 2 to 26.

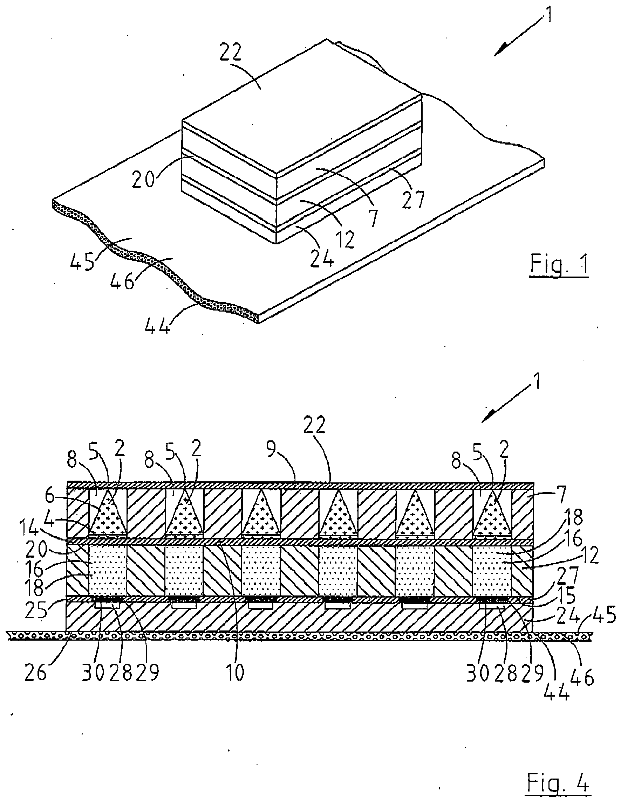

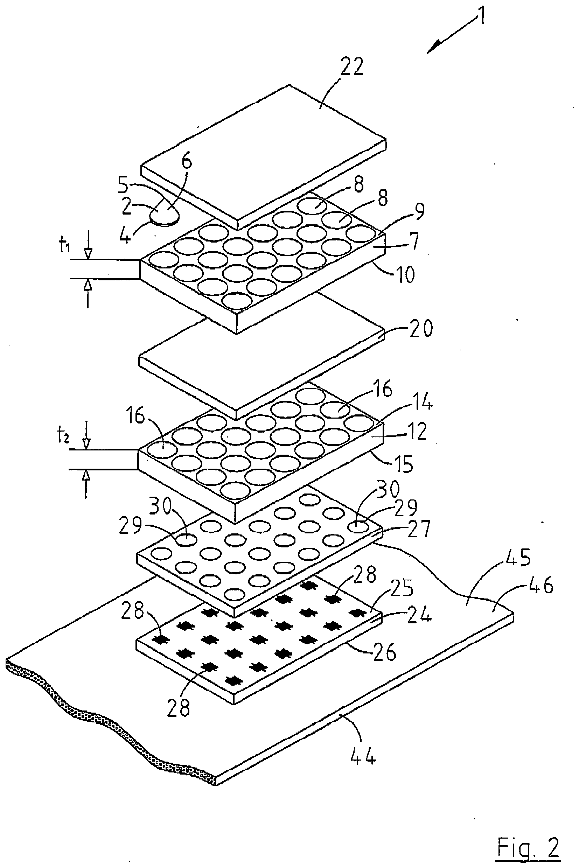

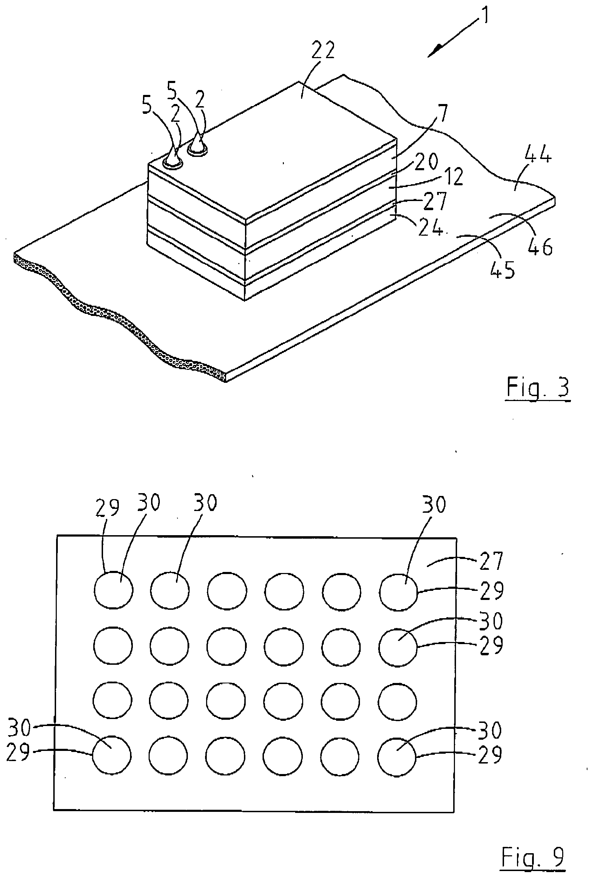

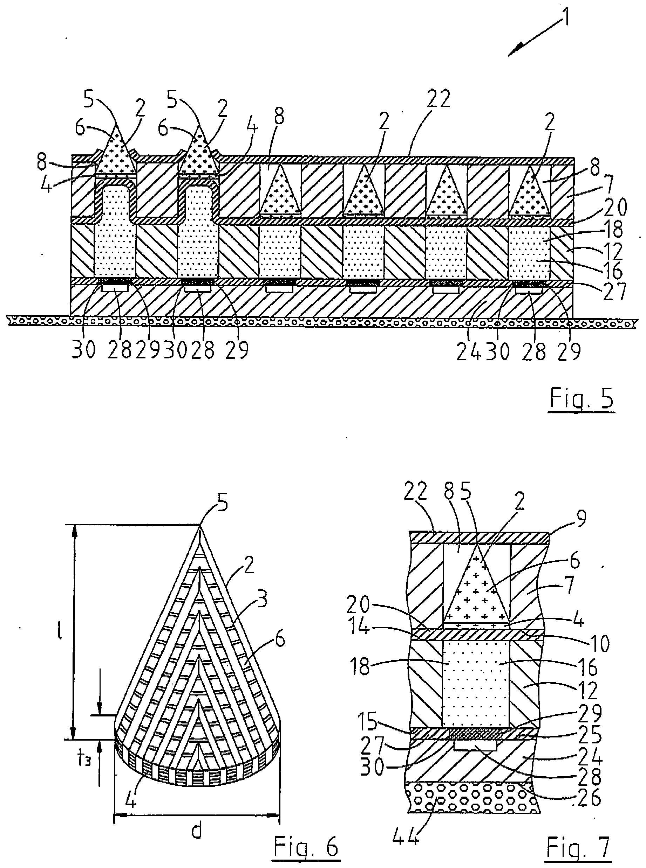

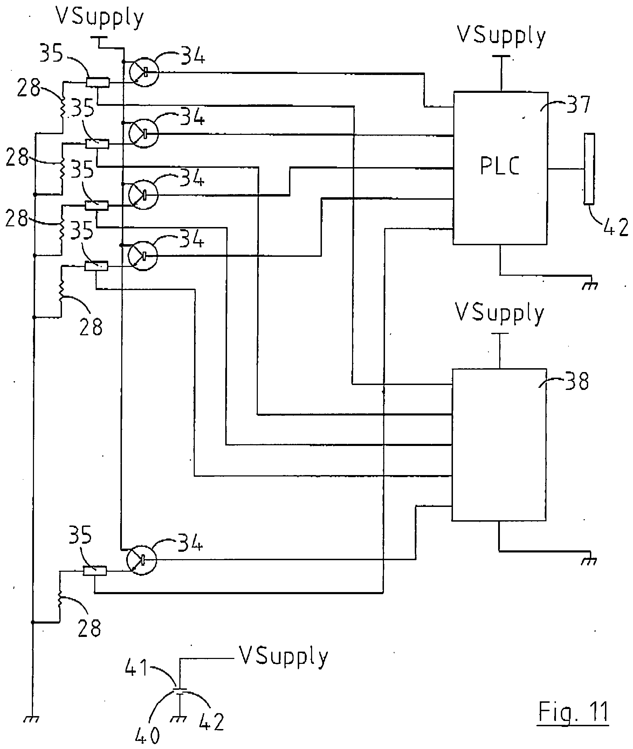

Referring to the drawings and initially to Figs. 1 to 11, there is illustrated a micro-dimensioned delivery device according to the invention, indicated generally by the reference numeral 1, for administering an active substance transdermally to a subject. The active substance is provided as an injectable element which is provided in the form of a solid form structure indicated generally by the reference numeral 2, and which in this example is formed by a scaffolding structure 3 of a biodegradable polymer material into which the active substance is impregnated. The scaffolding structure 3 is configured as a lattice structure of the biodegradable polymer material, which when impregnated with the active substance forms a substantially solid structure of circular transverse cross-section comprising a base portion 4 of constant transverse cross-section of diameter d, and a tapering portion 6 of conical shape extending axially from the base portion 4 and terminating in a pointed tip 5 for penetrating the skin of the subject. The lattice structure of the biodegradable material is constructed to facilitate slow release of the active substance. The active substance may be any medicament or other solution which it is desired to administer transdermally to a subject.

The device 1 is adapted for securing to the subject adjacent the site at which the active substance is to be administered to the subject, as will be described below.

The device 1 comprises a first housing provided by a first plate member 7 of a polymer material in which a plurality of first chambers 8 are formed for containing respective ones of the solid form structures 2 of the active substance. The first plate member 7 defines a first major surface 9 and an opposite second major surface 10. The first chambers 8 are formed by respective bores of circular transverse cross-section extending through the first plate member 7 from the first major surface 9 to the second major surface 10, and the diameter of the first chambers 8 and the diameter d of the base portion 4 of the solid form structures 2 are such that the base portion 4 of the solid form structures 2 are a sliding fit in the first chambers 8.

A second housing provided by a second plate member 12 also of a polymer material and having a first major surface 14 and a second major surface 15 is provided with a plurality of second chambers 16 also arranged in a matrix, and aligned with the first chambers 8 of the first plate member 7. The second chambers 16 are formed by respective second bores of circular transverse cross-section which extend through the second plate member 12 from the first major surface 14 to the second major surface 15. Each second chamber 16 houses an urging means, which in this embodiment of the invention is provided by a temperature responsive expandable driving substance 18, which is described below, for urging the solid form structures 2 through the corresponding first chambers 8 for penetrating the skin of the subject by the pointed tip 5, for in turn delivering the active substance transdermally to the subject.

A first membrane 20 of an expandable material, which is impermeable to both the active substance and the driving substance 18, is located between and sealably secured to the first plate member 7 and the second plate member 12 for sealably closing the adjacent ends of the first and second chambers 8 and 16 adjacent the second major surface 10 and the first major surface 14 of the first and second plate members 7 and 12, respectively, and for isolating the second chambers 16 from the corresponding first chambers 8. The expandability of the first membrane 20 is such as to permit expansion thereof into the first chambers 8 on expansion of the driving substance 18 in the corresponding second chambers 16, for in turn urging the corresponding solid form structures 2 through the first chambers 8 to penetrate the skin of the subject.

A second membrane 22 is sealably secured to the first major surface 9 of the first plate member 7 for sealably closing the ends of the first chambers 8 adjacent the first major surface 9 of the first plate member 7 so that the solid form structures 2 are maintained in the first chambers 8 in a sterile environment. The second membrane 22 is of a penetrable material which is penetrable by the pointed tips 5 of the solid form structures 2 as the solid form structures 2 are being urged through the first chambers 8 for in turn penetrating the skin of the subject.

A third plate member 24 having a first major surface 25 and a second major surface 26 is secured to the second plate member 12 with a third membrane 27 located therebetween. The third plate member 24 may be provided as a printed circuit board, or may be of a polymer material, and may be flexible or rigid. Alternatively, the third plate member 24 may be of a semiconductor material or a ceramics material. A plurality of activating means, provided by thin film resistor heating elements 28 are arranged in a matrix on the first major surface 25 of the third plate member 24 for raising the temperature of the expandable driving substance 18 in the respective second chambers 16 for expansion thereof. The heating elements 28 are aligned with the respective second chambers 16. The thin film resistors forming the heating elements 28 may be formed by any suitable process, which will be dependent on the material of the third plate member 24.

The third membrane 27 is sealably secured to the second major surface 15 of the second plate member 12 four sealably closing the ends of the second chambers 16 adjacent the second major surface 15. The third membrane 27 is of a heat insulating material, which is impermeable to the driving substance, and is provided with a matrix of openings 29 which are sealably closed by a plurality of heat conductive elements, namely, heat conductive discs 30 of metal material for facilitating heat transfer between the heating elements 28 and the driving substance 18 in the corresponding second chambers 16. The provision of the third membrane 27 as a heat insulating material minimises heat transfer between each heating element 28 and the second chambers 16 other than the corresponding adjacent second chamber 16, so that activation of each heating element 28 causes the driving substance in the corresponding second chamber 16 only to expand.

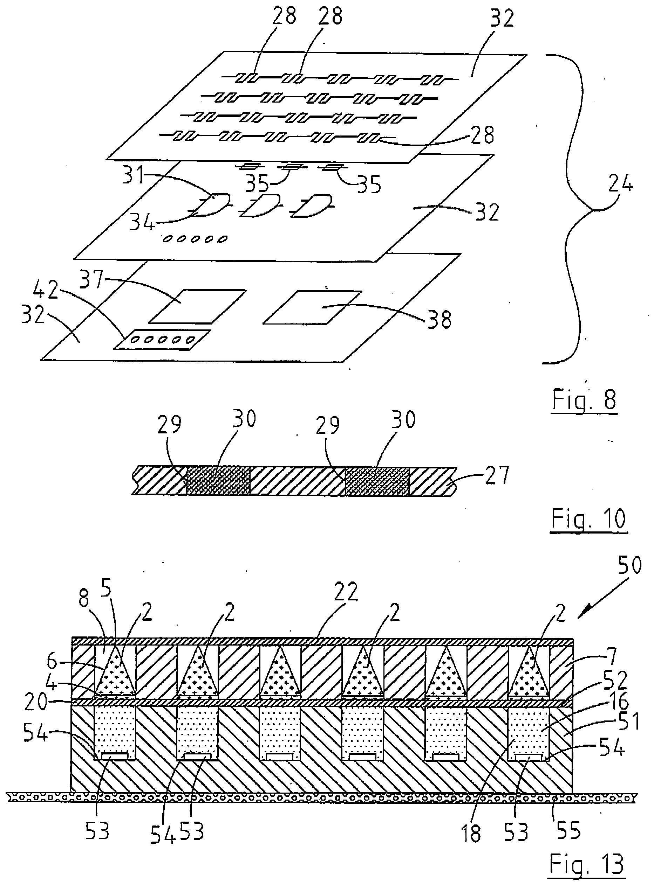

An electronic circuit 31 is also formed in or on the third plate member 24, and depending on whether the third plate member 24 is provided as a printed circuit board or as a semiconductor substrate, the circuit 31 may be formed on the first and/or second major surfaces 25 and 26, and/or within layers as will be understood by those skilled in the art. However, in a case where the third plate member 24 is provided as a semiconductor substrate, the circuit 31 may be formed as an integrated circuit on layers 32 of the substrate of the plate member 24, as illustrated in Fig. 8. The circuit 31 comprises a plurality of transistor switches 34, one transistor switch 34 being provided for each heating element 28 through which an electrical power supply is provided to the respective heating elements 28 for facilitating independent addressing of the respective heating elements 28. The heating elements 28 are powered through the transistors 34 and in turn through corresponding fuses 35 which are formed by thin film elements. Each thin film element which forms a fuse 35 is sized so that after conducting current to the corresponding heating element 28 for a predetermined time period, the fuse 35 goes into a permanent open circuit state, thereby preventing further activation of the corresponding heating element 28. The fuses 35 are rated so that the predetermined time period during which each fuse 35 conducts a current prior to going into an open circuit state is sufficient to raise the temperature of the driving substance 18 in the corresponding second chamber 16 for in turn urging the corresponding solid form structure 2 into penetrating engagement in the skin of a subject.

A programmable logic circuit 37 is also provided for operating the heating elements 28 in a desired sequence for urging the solid form structures 2 individually or in groups into penetrating engagement with the skin of the subject in accordance with a predefined treatment regime. For example, the programmable logic circuit 37 could be programmed to operate the heating elements 28 in groups, so that the respective groups would be sequentially operated at predefined time intervals over a predefined treatment period. The predefined time intervals could be such as to facilitate administration of a dose of the active substance three times per day, for example, in the morning, afternoon and evening, and the predefined treatment period could be a period over a number of days, for example, five days, seven days or the like. The number of heating elements 28 in each group would be dependent on the dose size, and in cases where the required dose size could be supplied by a single one of the solid form structures 2, the heating elements 28 would be individually operable. Depending on the material of the third plate member 24, the programmable logic circuit 37 may be formed as an integrated circuit on the third plate member 24, or may be provided separately for attachment to the device 1, for example, for attachment to the third plate member 24.

A monitoring circuit 38 is also provided for monitoring the state of the fuses 35 for in turn determining the first chambers 8 from which the solid form structures 2 have already been urged into penetration with the skin of the subject. Depending on the material of the third plate member 24, the monitoring circuit 38 would be provided in a similar manner to that in which the programmable logic circuit 37 is provided.

A power supply, provided by a battery 40, may be integrally formed with the delivery device 1 or independently thereof for coupling to the delivery device 1. In this example the battery 40 is formed in one or more of the first and/or second chambers 8 and 16 by placing an electrolyte in the appropriate ones of the first and/or second chambers 8 and 16. Suitable electrodes 41 are provided in the appropriate ones of the first and/or second chambers 8 and 16 to co-operate with the electrolyte to in turn provide electrical power to the integrated circuit 31 and to the heating elements 34 through the transistors 34 and the fuses 35. An input/output interface 42 is provided on the third plate member 24 for facilitating programming of the programmable logic control circuit 37. The input/output interface 42 may be adapted to facilitate wireless programming of the programmable logic circuit 37.

It is also envisaged that the programmable logic control circuit may be programmable to be responsive to externally applied signals, for example, an external signal from an external monitoring device, which would be provided for monitoring a particular characteristic of the subject. On the monitoring device indicating an abnormal situation, one or more of the heating elements 28 corresponding to one or more solid form structures 2 would be activated for urging those solid form structures 2 into penetrating engagement with the skin of the subject. The programmable logic circuit may also be programmable to be responsive to environmental sensors, which could be worn on the body of a subject, so that in the event of a change or an occurrence in the environment the heating elements 28 corresponding to appropriate ones of the solid form structures 2, which would be provided with a suitable active substance would be activated for administering the active substance to the subject. For example, such an environmental change could be an increase in the pollen count in the atmosphere, which would necessitate administering an antihistamine substance to the subject, and in which case the active substance in the appropriate ones of the solid form structures would be an antihistamine substance.

It is also envisaged that the device 1 may button operated manually by the subject. In which case, a manual button operated switch would be provided on the device 1, for example, on the third plate member 24, and the button switch would be coupled to the programmable logic circuit 37, so that on operating the button switch, the programmable logic circuit 37 would operate the appropriate one or ones of the heater elements 28 in order to activate the driving substance 18 for in turn urging the corresponding solid form structure or structures 2 to penetrate through the skin of the subject.

Returning now to the driving substance 18, the driving substance 18 may be any suitable solid, liquid or gas which has a relatively high coefficient of expansion. In this embodiment of the invention expansion of the driving substance is in response to a temperature increase, and is provided by a plurality of gas filled microspheres located in the respective second chambers 16. The microspheres are of the type supplied under the Trade Mark EXPANCEL. Further particulars of these microspheres are available on the website www.expancel.com. The microspheres are small gas filled spherical particles of plastics material. The shells of the microspheres are of a thermoplastic polymer-which softens in response to a rise in temperature, resulting.in a dramatic increase in the volume of the microspheres as the gas contained therein expands also in response to the rise in temperature. When in an unconfined space, such microspheres can expand to a volume which is forty times their original size.

Ideally, the driving substance should be such that expansion of the driving substance takes place relatively rapidly at a relatively low temperature. In the case of the gas filled microspheres, depending on their temperature rating, it has been found that by raising the temperature of the microspheres to temperatures in the range of 70°C to 130°C, adequate expansion is achieved for driving the corresponding solid form structure 2 through the corresponding first chamber 8 for penetrating the skin of the subject.

Alternatively, the driving substance may be a solid which on being subjected to heat converts directly to a gas, such as Azobisisobutylonitrile (AIBN). Needless to say, the driving substance may be a liquid which on being heated converts to a gas, or the driving substance may be a gas with a high coefficient of expansion.

As mentioned above, the delivery device 1 is of micro-dimensions, and the first plate member 7 is of thickness *t₁* of approximately 0.6mm, see Fig: 2. The second plate member 12 is of thickness *t₂* of approximately 1:0mm. Each first chamber 8 is of circular transverse cross-section and is of diameter of approximately 0.5mm, and each second chamber 16 is of circular transverse cross-section and of diameter of approximately 0.5mm. The base portion 4 of each solid form structure 2 is of constant circular transverse cross-section of diameter d just less than 0.5mm, so that the base portion 4 of each solid form structure 2 is a smooth sliding fit in the corresponding first chamber 8 from a position within the first chamber 8 to a position projecting through the second membrane 22 for penetrating the skin of a subject. The axial thickness *t₃* of the base portion 4 is approximately 0.07mm. The tapered portion 6 of each solid form structure 2 is of conical shape and the overall axial length *l* of each solid form structure 2, including the thickness *t₃* of the base portion 4 is approximately 0.5mm. The thickness of the third plate member 24 will depend on the material thereof, but typically, will be of the order of 2.0mm.

An adhesive patch 44 having an adhesive surface 45 is bonded to the second major surface 26 of the third plate member 24, and an outer peripheral portion 46 of the adhesive patch 44 is provided for bonding the adhesive patch 44 with the delivery device 1 attached thereto to the skin of a subject with the second membrane 22 abutting the skin of the subject.

In use, with the first, second and third plate members 7, 12 and 24 and the first and third membranes 20 and 27 assembled, and with the solid form structures 2 located in the first chambers 8 and the second membrane 22 sealably secured to the first major surface of the first plate member 7, and with the programmable logic control circuit 37 appropriately programmed, the delivery device 1 is attached to the skin of the subject at the appropriate site by the patch 44. At the appropriate programmed or otherwise timed predefined time intervals during the predefined treatment period, the appropriate one or ones of the heating elements 28 are powered up for expanding the driving substance 18 in the corresponding second chamber or chambers 16, for in turn urging the corresponding one or ones of the solid form structures 2 to penetrate through the second membrane 22, and in turn to penetrate the skin of the subject for transdermally delivering the active substance to the subject. Depending on the volume of the active substance to be administered to the subject in each dose, either one or an appropriate number of the heating elements are activated. On penetrating the skin of the subject, the scaffolding structure 3 of each solid form structure 2 may serve to facilitate a slow release of the active substance or otherwise from the corresponding solid form structure 2. Additionally, as the active substance is being slowly delivered to the subject, the scaffolding of the solid form structure gradually dissolves.

Thus, depending on the volume of the active substance to be administered to the subject in each dose, the heating elements 28 individually are sequentially activated at the appropriate predefined intervals over the predefined treatment period during which the doses of the active substance are to be administered to the subject, or alternatively, respective groups of the heating elements are sequentially activated at the appropriate predefined time intervals over the predefined treatment period.

It is envisaged that the solid form structures of the delivery device may each comprise the same active substance, or alternatively, respective groups of the solid form structures may comprise different active substances. This would facilitate the administration of more than one active substance to a subject over a predefined treatment period. Different ones of the active substances may be administered to the subject simultaneously, or at different times during the predefined treatment period.

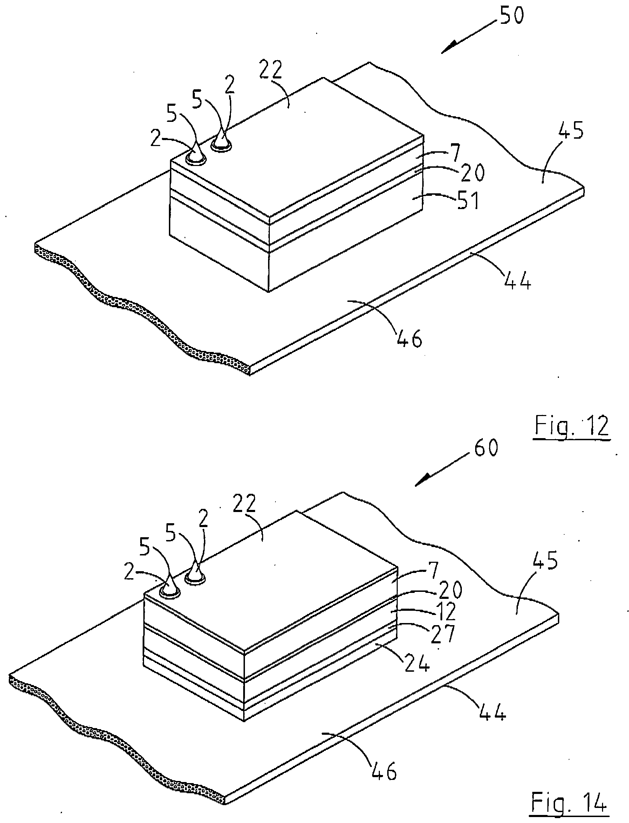

Referring now to Figs. 12 and 13, there is illustrated a micro-dimensioned delivery device according to another embodiment of the invention, indicated generally by the reference numeral 50 for administering an active substance transdemnally to a subject. The delivery device 50 is substantially similar to the delivery device 1, and similar components are identified by the same reference numerals. In this embodiment of the invention the second and third plate members are formed by a single integral plate member 51, which typically comprises a polymer material. The second chambers 16 are formed in the plate member 51 and extend into the plate member 51 from a first major surface 52 thereof. The activating means are provided by electrically powered heating elements 53 formed as thin film resistors in respective bases 54 of the corresponding second chambers 16. The heating elements 53 formed by thin film resistors, and are powered through an electronic circuit similar to the circuit 31 described with reference to the device 1. Wires (not shown) or other suitable electrically conductive tracks through the polymer material of the plate member 51 supply electrical power to the heating elements 53. If the plate member 51 is provided as a semiconductor substrate, the heating elements may be formed on the bases 54 of the second chambers 16 by a suitable integrated circuit forming process, for example, by chemical vapour deposition. Typically, an insulating layer, for example, a silicon oxide layer (not shown) would be provided over the heating elements 53. An integrated circuit (not shown) but substantially similar to the circuit 31 would also be provided in the plate member 51.

Otherwise, the delivery device 50 is substantially similar to the delivery device 1, and its use and operation are also similar to the delivery device 1.

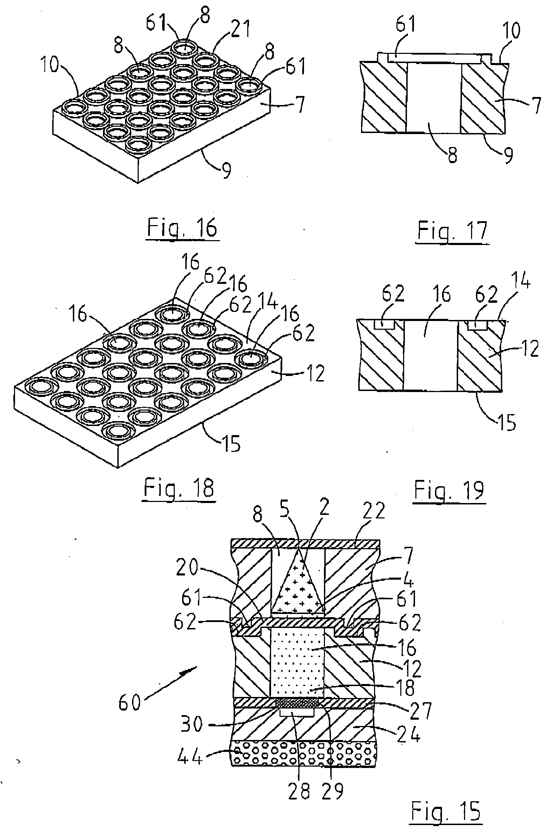

Referring now to Figs. 14 to 19, there is illustrated a micro-dimensional delivery device 60 according to another embodiment of the invention for administering an active substance transdermally to a subject. The delivery device 60 is substantially similar to the delivery device 1, and similar components are identified by the same reference numerals. In this embodiment of the invention a locating means is provided for locating the first and second plate members 7 and 12 relative to each other, so that the first and second chambers 8 and 16 are aligned with each other, and the locating means comprises respective pairs of interengageable complementary formations. One of the formations of each pair of formations comprises an annular projection 61 extending from the second major surface 10 of the first plate member 7, and the other of each pair of formations comprises a recess formed by an annular groove 62 extending into the first major surface 14 of the second plate member 12 for engaging the corresponding projection 61. Each annular projection 61 is of circular shape and extends from the second major surface 10 of the first plate member 7 completely around the corresponding first chamber 8 and slightly radially spaced apart therefrom. Each annular groove 62 extends completely around the corresponding second chamber 16 and slightly radially spaced apart therefrom. The annular projections 61 and the annular grooves 62 are dimensioned for accommodating the first membrane 20 therebetween as the first and second plate members 7 and 12 are brought into engagement with the first membrane for entrapping the first membrane between the annular projections 61 and the corresponding grooves 62.

Otherwise, the delivery device 60 is similar to the delivery device 1 and its use and operation are also similar to the delivery device 1.

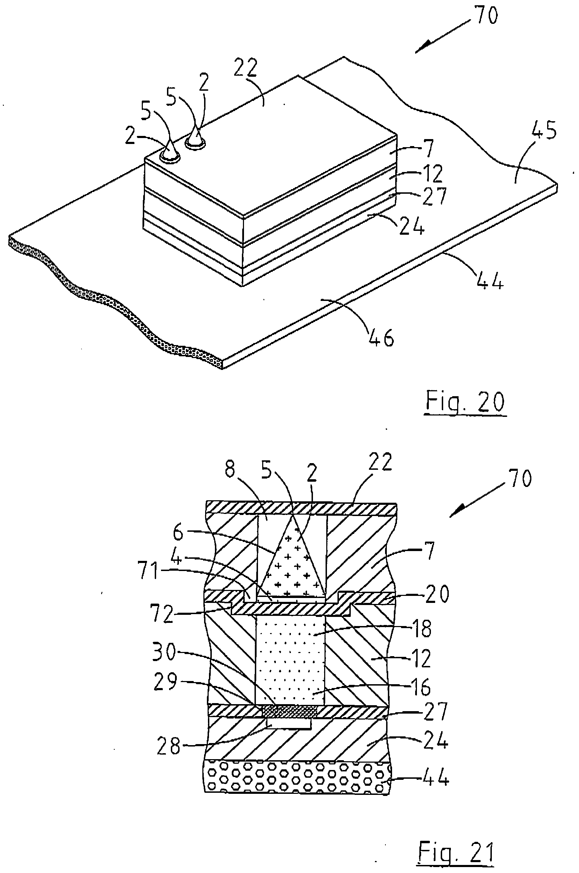

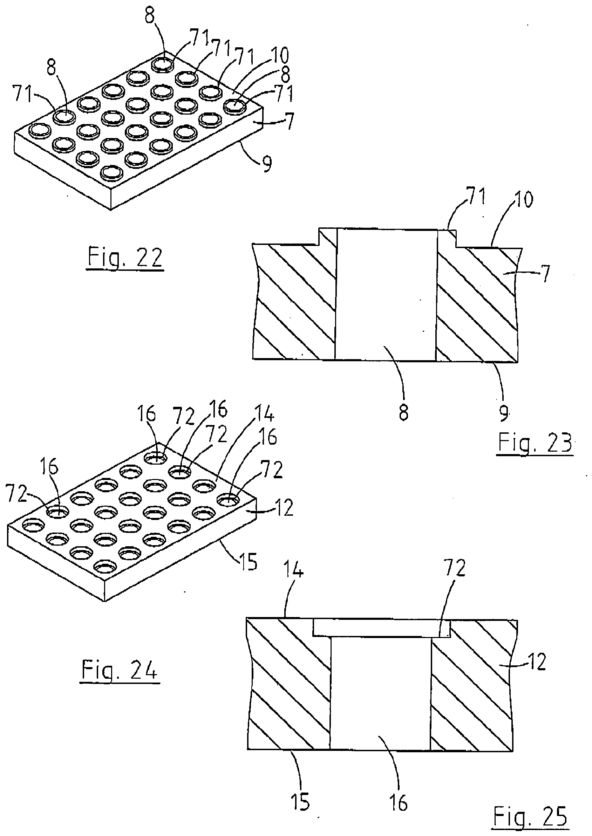

Referring now to Figs. 20 to 25, there is illustrated a micro-dimensioned delivery device 70 according to another embodiment of the invention for administering an active substance transdermally to a subject. The delivery device 70 is substantially similar to the delivery device 1, and similar components are identified by the same reference numerals. The main difference between the delivery device 70 and the delivery device 1 its in the provision of a locating means between the first and second plate members 7 and 12 for locating the first and second plate members 7 and 12 with the first and second chambers 8 and 16 aligned, In this embodiment of the invention the locating means comprises respective pairs of interengageable complementary formations, one of which formations is formed by an annular projection 71 extending from the second surface 10 of the first plate member 7 and a corresponding annular recess 72 formed in the first major surface 14 of the second plate member 12. The annular projections 71 are similar to the annular projections 61 of the delivery device 60. with the exception that the inner diameter of each annular projection 61 is similar to the diameter of the corresponding first chamber 8. In other words, the annular projections 71 are not spaced apart from the first chambers 8. The annular recesses 72 in this embodiment of the invention are formed into the second chambers 16 adjacent the first major surface 12 of the second plate member 12. The annular recesses 72 and the projections 71 are dimensioned for accommodating the first membrane 20 therebetween when the first and second plate members 7 and 12 are brought into engagement with each other with the first membrane 20 entrapped by the co-operating action of the annular projections 71 with the corresponding annular recesses 72.

Otherwise, the delivery device 70 and its use and operation are similar to the delivery device 1.

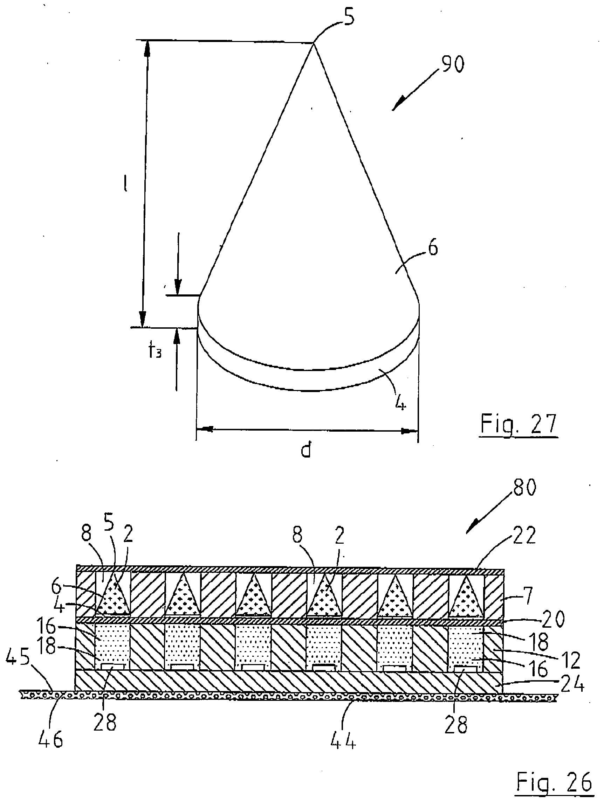

Referring now to Fig. 26, there is illustrated a micro-dimensioned delivery device 80 according to another embodiment of the invention for administering an active substance transdermally to a subject. The delivery device 80 is substantially similar to the delivery device 1, and similar components are identified by the same reference numerals. The main difference between the delivery device 80 and the delivery device 1 is that in this embodiment of the invention the third membrane has been omitted, and the third plate member 24 is provided as a flexible polymer sheet which is sealably secured to the second major surface 15 of the second plate member 12 for sealing the second chambers 16. Electrical heating elements 28, which are formed by thin film resistors, are formed on the first major surface 25 of the third plate member 24. An electronic circuit (not shown, but similar to the circuit 31 of the device 1) is provided on the second major surface 26 of the third plate member 24. Electrically conductive wires or suitable electrically conductive tracks couple the heating elements 28 to the circuit provided on the second major surface 26 of the third plate member 24 through vias (not shown) through the third plate member 24.

Otherwise, the delivery device 80 and its use and operation is similar to the delivery device 1.

Referring now to Fig. 27, there Is Illustrated an injectable element which is provided by a solid form structure indicated generally by the reference numeral 90, for use in any of the delivery devices 1, 50, 60, 70 or 80 already described. In this embodiment of the invention the solid form structure 90 comprises a mixture of an active substance and an excipient. The active substance and the excipient are mixed together with an appropriate binder to set to form the solid form structure in the form of a solid. In this embodiment of the invention a lattice scaffolding structure is not required.

It is also envisaged that when preparing the injectable elements, whether they be prepared in the form of the solid form structure 2 or in the form of the solid form structure 90, the injectable element may be prepared with the active substance located in the tapering portion 6 towards the tip 5, in order to ensure that when the solid form structure penetrates through the skin of the subject to the site beneath the skin of the subject, the active substance is concentrated at the appropriate site. In which case, the remainder of the tapering portion 6 and the base portion 4 would not include any active substance.

It is envisaged that in cases where the programmable logic control circuit is programmable in a wireless manner, the electronic circuit 31 of the relevant devices would be provided with wireless connectivity and would be programmable by, for example, Bluetooth.

In certain cases, it is envisaged that the power to the delivery device may be supplied by kinetic movement, as for example in-an automatic watch, whereby the kinetic movement may be converted to electrical energy for charging a battery, or for charging a capacitor or other suitable electrical energy storing device.

While the urging means for urging the solid form structures 2 to penetrate the skin of the subject has been described as being provided by gas filled microspheres sold under the Trade Mark EXPANCEL, any other suitable gas filled microspheres may be used. Needless to say, other suitable driving substance may be used, and such other driving substances may, for example, be an expandable liquid, an expandable solid or an expandable gas. It is also envisaged that the driving substance may be a solid which would convert directly from the solid phase to the gaseous phase. Additionally, it is envisaged that other suitable urging means besides a driving fluid may be used. In certain cases, it is envisaged that the driving substance may comprise two chemicals, such that when mixed, the chemicals would expand to urge the first membrane into the corresponding first chamber. In which case, the two chemicals would be maintained separated from each other until the corresponding solid form structure is to be urged from the corresponding first chamber. The two chemical substances could be maintained separated from each other by a suitable membrane, which would be burstable by a suitable activating means.

Needless to say, while the driving substance has been described as being provided by gas filled microspheres which expand at a relatively low temperature, in certain cases, it is envisaged that a driving substance which converted from the liquid phase to a gaseous phase at a higher temperature, and indeed, at a relatively high temperature, may also be suitable.

Further, it is envisaged that the driving substance may be a porous material which would be impregnated with a gas of high co-efficient of expansion or a liquid which would convert to a gaseous phase at an appropriate temperature, and when subjected to the appropriate temperature the gas would expand or the liquid would convert to the gaseous phase. The expanding gas would cause expansion of the porous material, which would in turn urge the first membrane into the corresponding first chamber. Alternatively, the expanding gas may expand out of the porous material to directly act on the first membrane to urge the first membrane into the corresponding first chamber.

Additionally, each urging means may be provided by a piston which would be sealably located in the corresponding second chamber, and would be sealably slideable into and through the corresponding first chamber in response to expansion of a driving substance. It is also envisaged that the solid form structures may be sealably slideable longitudinally in the first chambers, and the driving substance would act directly on the solid form structures for urging the solid form structures through the first chambers for penetrating the skin of the subject.

While the delivery devices have been described for administering an active substance transdermally to a subject, the delivery devices may be adapted for delivering an active substance to a subject subcutaneously, intradermally, or to any other depth beneath the skin of the subject This would be achieved by providing the solid form structures to be of an appropriate axial length.

Additionally, it will be appreciated that while the active substance has been described as being incorporated in a solid form structure which is formed by a scaffolding structure of a biodegradable polymer material, the scaffolding structure may be provided by any suitable type of biodegradable lattice structure, indeed, a non-biodegradable material, polymer or otherwise, or indeed any other suitable biocompatible material may be used for forming a lattice structure which would be suitable for being impregnated with the active substance. Additionally, in certain cases, it is envisaged that the active substance may itself be formed into the solid structure without any other ingredients or components in the structure, or the active substance may be formed into the solid form structure in a mixture comprising only the active substance and an excipient as described with reference to Fig. 27.

It is also envisaged that the support material of each solid form structure may be porous, and the active substance would be impregnated therein.

Alternatively, the active substance may be coated onto the support material of each solid form structure.

It is also envisaged that the support material of each solid form structure may be adapted for facilitating slow release of the active substance therefrom.

Additionally it is envisaged that each solid form structure may be slideable in the corresponding first chamber from a position entirely within the first chamber to a position whereby at least a portion of the solid form structure extends outwardly of the first chamber for penetrating the skin of the subject.

It is envisaged that each solid form structure may be a sealable sliding fit within the corresponding first chamber, and the urging means would act directly on the solid form structure for urging the solid form structure through the first chamber.

It is also envisaged that the maximum transverse cross-sectional dimension of each solid form structure would lie in the range of 0.1 mm to 2mm, and that the axial length of each solid form structure would lie in the range of 0.1 mm to 2mm.

It is also envisaged that the delivery devices may be supplied without the solid form structures, and in which case, the solid form structures would be placed in the first chambers subsequently, which would then be sealed with the second membrane. The subsequent assembly of the solid form structures into the first chambers could be carried out by a user of the device, or alternatively, could be carried out under factory sterile conditions.

It is also envisaged that the second membrane may be omitted or may be provided to be peeled off just prior to use, so that the first major surface of the first plate member would be in direct engagement with the skin of the subject.

It is also envisaged that the solid form structures may be bonded to the first membrane, and in which case, it is envisaged that the driving substance in the second chambers would be of a type that once expanded would remain in the expanded state.

It will be appreciated that the delivery devices according to the invention may be of any size, and may comprise any number of first and second chambers and any number of solid form structures. In general, the size and the number of the first and second chambers will depend on the size and number of the solid form structures. Additionally, the number of solid form structures will be determined by the number of solid form structures required to provide each dose of the active substance, and the number of doses of the active substance in a treatment regime. Needless to say, while the first and second chambers and the solid form structures have been described as being of specific dimensions, the first and second chambers and the solid form structures may be of any suitable dimensions.

While the delivery devices have been described as having a second chamber corresponding to each first chamber, it is envisaged in certain cases that a number of first chambers may be provided to a single chamber. For example, in cases where a single dose of the active substance requires a number of solid form structures to be simultaneously delivered from the first chambers, a single second chamber may be provided corresponding to the appropriate number of first chambers in order to supply the single dose of the active substance.

While the activating means have been described as being specific types of electrically powered heating elements, any suitable type of heating elements may be used. Additionally, other suitable types of activating means besides heating elements may be used. In general, it is envisaged that the number of activating means will be similar to the number of second chambers, although, not necessarily.

It is also envisaged that the third plate member may comprise a material selected from one of silicon, ceramics, polyimide and FR4.

## Claims

1. A delivery device for administering an active substance to a subject in the form of a solid form structure (2,90), the device comprising a first housing (7) having a plurality of first chambers (8) located therein adapted to accommodate respective ones of the solid form structures (2,90), a second housing (12,51) having a plurality of second chambers (16), and a plurality of urging means (18) located in the respective second chambers (16) for urging the solid form structures (2,90) through the first chambers (8) to penetrate the skin of the subject, **characterised in that** a first membrane (20) Is located between the first and second chambers (8,16) for sealably isolating each first chamber (8) from the second chambers (16), and the first membrane (20) is deformable into each first chamber (8) under the action of the corresponding urging means (18) for urging the corresponding solid form structure (2,90) through the first chamber (8).

2. A delivery device as claimed in Claim 1 **characterised In that** one second chamber (16) is provided corresponding to each first chamber (8), the second chambers (16) being aligned with the respective corresponding first chambers (8).

3. A delivery device as claimed In Claim 1 or 2 **characterised In that** the first membrane (20) comprises a material Impermeable to the urging means (18), and to the active substance (2,90),

4. A delivery device as claimed in any preceding claim **characterised in that** the first housing (7) comprises a first plate member (7) defining opposite first and second major surfaces (9.10), and each first chamber (8) is formed by a corresponding first bore (8) extending through the first plate member (7) from the first major surface (9) thereof to the second major surface (10).

5. A delivery device as claimed in Claim 4 **characterised in that** the second housing (12,51) comprises a second plate member (12) having opposite first and second major surfaces (14,15), and each second chamber (16) Is formed by a corresponding second bore (16) extending through the second plate member (12) from the first major surface (14) thereof to the second major surface (15).

6. A delivery device as claimed in Claim 5 **characterised in that** a plurality of pairs of interengageable complementary formations (61,62,71,72) are provided for locating the first and second plate members (7,12,51) relative to each other with each first chamber (8) aligned with the corresponding second chamber (18), one of the interengageable complementary formations (61,71) of each pair thereof being located on the second major surface (10) of the first plate member (7) adjacent a corresponding one of the first chambers (8), and the other one (62,72) of the interengageable complementary formations of each pair thereof being located on the first major surface (14) of the second plate member (12,51), the interengageable complementary formations (61,62,71,72) of the respective pairs thereof being co-operable with each other for entrapping the first membrane (20) therebetween.

7. A delivery device as claimed in any of Claims 4 to 6 **characterised in that** a second membrane (22) is provided for sealably closing each first chamber (8) adjacent the first major surface (9) of the first plate member (7), the second membrane (22) being adapted to be penetrable by a pointed tip (5) of each solid form structure (2,90).

8. A delivery device as claimed In any preceding claim **characterised in that** at least one activating means (28) is provided for activating the urging means (18) to urge at least one of the solid form structures (2,90) through the corresponding first chamber (8) to penetrate the skin of a subject.

9. A delivery device as claimed in Claim 8 **characterised in that** a plurality of activating means (28) are provided, each activating means (28) being provided for activating the urging means (18) In a corresponding one of the second chambers (16).

10. A delivery device as claimed in Claim 9 **characterised in that** the respective activating means (28) are aligned with corresponding ones of the second chambers (16).

11. A delivery device as claimed in any of Claims 8 to 10 **characterised in that** each activating means (28) comprises a heating means (28).

12. A delivery device as claimed in any of Claims 8 to 11 **characterised In that** a third membrane (27) is located between the activating means (28) and the second chambers (16) for sealably closing the second chamber (18), the third membrane (27) having a plurality of openings (29) formed therethrough adjacent respective corresponding ones of the second chambers (16), each opening (29) being sealably closed by a corresponding heat conducting element (30) for conducting heat to the urging means (18) in the corresponding second chamber (16).

13. A delivery device as claimed in any preceding claim **characterised in that** the first housing (7) comprises a plurality of solid form structures (2,90), one solid form structure (2,90) being located in each first chamber (8).

14. A delivery device as claimed in any preceding claim **characterised in that** the urging means (18) comprises an expandable driving substance in the form of one of
- a liquid which is responsive to one of temperature change and chemical activation for converting from a liquid phase to a gaseous phase,
- a solid which is responsive to one of temperature change and chemical activation for transitioning directly from a solid phase to a gaseous phase,
- a plurality of gas filled microspheres which are responsive to temperature change for expansion thereof, and
- a porous material, the pores of which are gas filled.

## Patentansprüche

1. Abgabevorrichtung zum Verabreichen eines aktiven Wirkstoffes an ein Subjekt in Form einer Struktur (2,90) in fester Form, wobei die Vorrichtung ein erstes Gehäuse (7) mit einer Mehrzahl von darin angeordneten ersten Kammern (8), die dafür ausgebildet sind, entsprechende der Strukturen (2,90) in fester Form aufzunehmen, ein zweites Gehäuse (12,51) mit einer Mehrzahl von zweiten Kammern (16) und einer Mehrzahl von in den entsprechenden zweiten Kammern (16) angeordneten Druckeinrichtungen (18) zum Drücken der Strukturen (2,90) in fester Form durch die ersten Kammern (8), so dass sie die Haut des Subjekts durchdringen, umfasst, **dadurch gekennzeichnet, dass** eine erste Membran (20) zwischen der ersten und der zweiten Kammer (8,16) zum abdichtbaren Trennen jeder ersten Kammer (8) von den zweiten Kammern (16) angeordnet ist und die erste Membran (20) unter Einwirkung der entsprechenden Druckeinrichtung (18) zum Drücken der entsprechenden Struktur (2,90) in fester Form durch die erste Kammer (8) in jede erste Kammer (8) verformbar ist.

2. Abgabevorrichtung nach Anspruch 1, **dadurch gekennzeichnet, dass** eine zweite Kammer (16) entsprechend jeder ersten Kammer (8) vorgesehen ist, wobei die zweiten Kammern (16) in Ausrichtung mit den entsprechenden ersten Kammern (8) angeordnet sind.

3. Abgabevorrichtung nach Anspruch 1 oder 2, **dadurch gekennzeichnet, dass** die erste Membran (20) ein Material umfasst, das von der Druckvorrichtung (18) und dem aktiven Wirkstoff (2,90) nicht durchdrungen werden kann.

4. Abgabevorrichtung nach einem der vorhergehenden Ansprüche, **dadurch gekennzeichnet, dass** das erste Gehäuse (7) ein erstes Plattenelement (7) umfasst, das eine erste und eine zweite Hauptfläche (9,10) definiert, die einander gegenüber angeordnet sind, und jede erste Kammer (8) von einer entsprechenden ersten Bohrung (8) gebildet ist, die sich durch das erste Plattenelement (7) von dessen erster Hauptfläche (9) zur zweiten Hauptfläche (10) erstreckt.

5. Abgabevorrichtung nach Anspruch 4, **dadurch gekennzeichnet, dass** das zweite Gehäuse (12, 51) ein zweites Plattenelement (12) mit einer ersten und einer zweiten Hauptoberfläche (14,15), die einander gegenüber angeordnet sind, umfasst und jede zweite Kammer (16) durch eine entsprechende zweite Bohrung (16) gebildet ist, die sich durch das zweite Plattenelement (12) von dessen erster Hauptfläche (14) zur zweiten Hauptfläche (15) erstreckt.

6. Abgabevorrichtung nach Anspruch 5, **dadurch gekennzeichnet, dass** eine Mehrzahl von Paaren von komplementären Ausbildungen (61,62,71,72), die ineinander eingreifen können, vorgesehen ist, um die ersten und die zweiten Plattenelemente (7,12,51) relativ zueinander zu positionieren, wobei jede erste Kammer (8) in Ausrichtung mit der entsprechenden zweiten Kammer (16) angeordnet ist, wobei eine der ineinander eingreifbaren komplementären Ausbildungen (61,71) jedes Paares davon an der zweiten Hauptfläche (10) des ersten Plattenelements (7) angeordnet ist, benachbart einer entsprechenden der ersten Kammern (8), und die andere (62,72) der ineinander eingreifbaren komplementären Ausbildungen jedes Paares davon an der ersten Hauptfläche (14) des zweiten Plattenelementes (12,51) angeordnet ist, wobei die ineinander eingreifbaren komplementären Ausbildungen (61,62,71,72) der entsprechenden Paare davon gemeinsam wirken können, um die erste Membran (20) dazwischen festzuhalten.

7. Abgabevorrichtung nach einem der Ansprüche 4 bis 6, **dadurch gekennzeichnet, dass** eine zweite Membran (22) zum abdichtbaren Verschließen jeder ersten Kammer (8) benachbart der ersten Hauptfläche (9) des ersten Plattenelements (7) vorgesehen ist, wobei die zweite Membran (22) dafür ausgebildet ist, dass sie von einer spitz zulaufenden Spitze (5) jeder Struktur (2, 90) in fester Form durchdrungen werden kann.

8. Abgabevorrichtung nach einem der vorhergehenden Ansprüche, **dadurch gekennzeichnet, dass** mindestens eine Aktivierungseinrichtung (28) zum Aktivieren der Druckeinrichtung (18) vorgesehen ist, um mindestens eine der Strukturen (2, 90) in fester Form durch die entsprechende erste Kammer (8) zu drücken, um die Haut eines Subjekts zu durchdringen.

9. Abgabevorrichtung nach Anspruch 8, **dadurch gekennzeichnet, dass** eine Mehrzahl von Aktivierungseinrichtungen (28) vorgesehen ist, wobei jede Aktivierungseinrichtung (28) zum Aktivieren der Druckeinrichtung (18) in einer entsprechenden der zweiten Kammern (16) vorgesehen ist.

10. Abgabevorrichtung nach Anspruch 9, **dadurch gekennzeichnet, dass** die entsprechende Aktivierungseinrichtung (28) in Ausrichtung mit entsprechenden der zweiten Kammern (16) angeordnet ist.

11. Abgabevorrichtung nach einem der Ansprüche 8-10, **dadurch gekennzeichnet, dass** jede Aktivierungseinrichtung (28) eine Wärmeeinrichtung (28) umfasst.

12. Abgabevorrichtung nach einem der Ansprüche 8 bis 11, **dadurch gekennzeichnet, dass** eine dritte Membran (27) zwischen der Aktivierungseinrichtung (28) und den zweiten Kammern (16) zum abdichtbaren Verschließen der zweiten Kammer (18) angeordnet ist, wobei die dritte Membran (27) eine Mehrzahl von dadurch gebildeten Öffnungen (29), entsprechenden der zweiten Kammern (16) benachbart angeordnet, aufweist, wobei jede Öffnung (29) abdichtbar durch ein entsprechendes Wärmeleitelement (30) zum Leiten von Wärme zu der Druckeinrichtung (18) in der entsprechenden zweiten Kammer (16) verschlossen ist.

13. Abgabevorrichtung nach einem der vorhergehenden Ansprüche, **dadurch gekennzeichnet, dass** das erste Gehäuse (7) eine Mehrzahl von Strukturen (2,90) in fester Form umfasst, wobei eine Struktur (2,90) in fester Form in jeder ersten Kammer (8) angeordnet ist.

14. Abgabevorrichtung nach einem der vorhergehenden Ansprüche, **dadurch gekennzeichnet, dass** die Druckeinrichtung (18) einen ausdehnbaren Treiberwirkstoff in der Form eines der folgenden Stoffe hat:
- eine Flüssigkeit, die auf eines von Temperaturänderung und chemischer Aktivierung zum Umwandeln aus einer flüssigen Phase in eine gasförmige Phase reagiert,
- ein Feststoff, der auf eines einer Temperaturänderung und chemischer Aktivierung zum direkten Übergang aus einer festen Phase in eine gasförmige Phase reagiert,
- eine Mehrzahl von gasgefüllten Mikrokugeln, die auf Temperaturänderung für deren Ausdehnung ansprechen, und
- ein poröses Material, dessen Poren mit Gas gefüllt sind.

## Revendications

1. Dispositif de distribution pour administrer une substance active à un patient sous la forme d'une structure de forme solide (2, 90), le dispositif comprenant un premier boîtier (7) ayant une pluralité de premières chambres (8) positionnées à l'intérieur de ce dernier, adaptées pour loger des structures respectives des structures de forme solide (2, 90), un second boîtier (12, 51) ayant une pluralité de secondes chambres (16), et une pluralité de moyens de poussée (18) positionnés dans les secondes chambres (16) respectives pour pousser les structures de forme solide (2, 90) à travers les premières chambres (8) pour pénétrer dans la peau du patient, **caractérisé en ce qu'**une première membrane (20) est positionnée entre les première et seconde chambres (8, 16) pour isoler, de manière étanche, chaque première chambre (8) des secondes chambres (16), et la première membrane (20) est déformable dans chaque première chambre (8) sous l'action des moyens de poussée (18) correspondants pour pousser la structure de forme solide (2, 90) correspondante à travers la première chambre (8).

2. Dispositif de distribution selon la revendication 1, **caractérisé en ce qu'**une seconde chambre (16) est prévue en correspondance de chaque première chambre (8), les secondes chambres (16) étant alignées avec les premières chambres (8) correspondantes respectives.

3. Dispositif de distribution selon la revendication 1 ou 2, **caractérisé en ce que** la première membrane (20) comprend un matériau imperméable aux moyens de poussée (18) et à la substance active (2, 90).

4. Dispositif de distribution selon l'une quelconque des revendications précédentes, **caractérisé en ce que** le premier boîtier (7) comprend un premier élément de plaque (7) définissant des première et seconde surfaces majeures (9, 10) opposées, et chaque première chambre (8) est formée par un premier alésage (8) correspondant s'étendant à travers le premier élément de plaque (7) à partir de sa première surface majeure (9) jusqu'à la seconde surface majeure (10).

5. Dispositif de distribution selon la revendication 4, **caractérisé en ce que** le second boîtier (12, 51) comprend un second élément de plaque (12) ayant des première et seconde surfaces majeures (14, 15) opposées, et chaque seconde chambre (16) est formée par un second alésage (16) correspondant s'étendant à travers le second élément de plaque (12) à partir de sa première surface majeure (14) jusqu'à la seconde surface majeure (15).

6. Dispositif de distribution selon la revendication 5, **caractérisé en ce qu'**une pluralité de paires de formations complémentaires interchangeables (61, 62, 71, 72) sont prévues pour positionner les premier et second éléments de plaque (7, 12, 51) les uns par rapport aux autres avec chaque première chambre (8) alignée avec la seconde chambre (16) correspondante, l'une des formations complémentaires interchangeables (61, 71) de chacune de ses paires étant positionnée sur la seconde surface majeure (10) du premier élément de plaque (7) adjacente à une chambre correspondante des premières chambres (8), et l'autre formation (62, 72) des formations complémentaires interchangeables de chacune de ses paires étant positionnée sur la première surface majeure (14) du second élément de plaque (12, 51), les formations complémentaires interchangeables (61, 62, 71, 72) de leurs paires respectives pouvant coopérer entre elles pour piéger la première membrane (20) entre elles.

7. Dispositif de distribution selon l'une quelconque des revendications 4 à 6, **caractérisé en ce qu'**une deuxième membrane (22) est prévue pour fermer de manière étanche chaque première chambre (8) adjacente à la première surface majeure (9) du premier élément de plaque (7), la deuxième membrane (22) étant adaptée pour pouvoir être pénétrée par une pointe pointue (5) de chaque structure de forme solide (2, 90).

8. Dispositif de distribution selon l'une quelconque des revendications précédentes, **caractérisé en ce qu'**au moins un moyen d'activation (28) est prévu pour activer les moyens de poussée (18) afin de pousser au moins l'une des structures de forme solide (2, 90) à travers la première chambre (8) correspondante afin de pénétrer dans la peau d'un patient.

9. Dispositif de distribution selon la revendication 8, **caractérisé en ce qu'**une pluralité de moyens d'activation (28) sont prévus, chaque moyen d'activation (28) étant prévu pour activer les moyens de poussée (18) dans une chambre correspondante des secondes chambres (16).

10. Dispositif de distribution selon la revendication 9, **caractérisé en ce que** les moyens d'activation (28) respectifs sont alignés avec les chambres correspondantes des secondes chambres (16).

11. Dispositif de distribution selon l'une quelconque des revendications 8 à 10, **caractérisé en ce que** chaque moyen d'activation (28) comprend un moyen de chauffage (28).

12. Dispositif de distribution selon l'une quelconque des revendications 8 à 11, **caractérisé en ce qu'**une troisième membrane (27) est positionnée entre les moyens d'activation (28) et les secondes chambres (16) pour fermer de manière étanche la seconde chambre (16), la troisième membrane (27) ayant une pluralité d'ouvertures (29) formées à travers des chambres correspondantes respectives adjacentes des secondes chambres (16), chaque ouverture (29) étant fermée de manière étanche par un élément conducteur de chaleur (30) correspondant pour conduire la chaleur jusqu'aux moyens de poussée (18) dans la seconde chambre (16) correspondante.

13. Dispositif de distribution selon l'une quelconque des revendications précédentes, **caractérisé en ce que** le premier boîtier (7) comprend une pluralité de structures de forme solide (2, 90), une structure de forme solide (2, 90) étant positionnée dans chaque première chambre (8).

14. Dispositif de distribution selon l'une quelconque des revendications précédentes, **caractérisé en ce que** le moyen de poussée (18) comprend une substance d'entraînement expansible se présentant sous la forme de l'un parmi
- un liquide qui est sensible à l'un parmi le changement de température et l'activation chimique pour passer d'une phase liquide à une phase gazeuse,
- un solide qui est sensible à l'un parmi le changement de température et l'activation chimique pour effectuer une transition directement d'une phase solide à une phase gazeuse,
- une pluralité de microsphères remplies de gaz qui sont sensibles au changement de température pour leur dilatation, et
- un matériau poreux, dont les pores sont remplis de gaz.
